(19) Europäisches Patentamt / European Patent Office / Office européen des brevets

(11) EP 4 430 961 A2

(12) EUROPEAN PATENT APPLICATION

(43) Date of publication:
**18.09.2024 Bulletin 2024/38**

(21) Application number: **24192713.6**

(22) Date of filing: **06.11.2015**

(51) International Patent Classification (IPC):
***A24B 15/16*** *(2020.01)* ***A24F 40/10*** *(2020.01)*
***A24F 40/42*** *(2020.01)* ***A24F 47/00*** *(2020.01)*
***A24B 15/167*** *(2020.01)*

(52) Cooperative Patent Classification (CPC):
**A24B 15/16; A24B 15/167; A24F 40/42;**
A24F 40/10

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.11.2014 GB 201419866**

(62) Document number(s) of the earlier application(s) in accordance with Art. 76 EPC:
**20183945.3 / 3 738 447**
**18212381.0 / 3 491 941**
**15794254.1 / 3 214 957**

(71) Applicant: **Nicoventures Trading Limited**
**London**
**Greater London WC2R 3LA (GB)**

(72) Inventors:
- **MCADAM, Kevin Gerard**
  **London, WC2R 3LA (GB)**
- **BRUTON, Connor**
  **London, WC2R 3LA (GB)**
- **TRANI, Marina**
  **London, WC2R 3LA (GB)**

(74) Representative: **D Young & Co LLP**
**3 Noble Street**
**London EC2V 7BQ (GB)**

Remarks:
This application was filed on 02-08-2024 as a divisional application to the application mentioned under INID code 62.

# (54) ELECTRONIC VAPOUR PROVISION SYSTEM

(57) There is provided an electronic vapour provision system comprising: a vaporiser for vaporising liquid for inhalation by a user of the electronic vapour provision system; a power supply comprising a cell or battery for supplying power to the vaporiser a container in which is contained a nicotine solution, wherein at least 60 wt% of the nicotine present in the solution is in protonated form, and wherein at least a portion of the container in contact with the nicotine solution is formed from polycarbonate or polypropylene.

EP 4 430 961 A2

**Description**

**FIELD OF THE INVENTION**

The present disclosure relates to containers which contain a nicotine solution and to electronic vapour provision systems such as electronic nicotine delivery systems (e.g. e-cigarettes) incorporating such containers.

**BACKGROUND TO THE INVENTION**

Electronic vapour provision systems such as e-cigarettes generally contain a reservoir of liquid which is to be vaporised, typically containing nicotine. When a user inhales on the device, a heater is activated to vaporise a small amount of liquid, which is therefore inhaled by the user.

The use of e-cigarettes in the UK has grown rapidly, and it has been estimated that there are now over a million people using them in the UK.

The liquid to be vaporised in e-cigarettes is typically a solution containing nicotine. The solvent may be, for example, glycerol. The vaporising part of the device is often designed for multiple uses, although single use devices do exist. In multiple and single use devices a container holding the nicotine solution is present. The container is stored for significant periods from the time of filling until use. This period includes the time of distribution, stocking by a retailer and storage by the end user before use. During this storage period, loss of nicotine content may occur.

**SUMMARY OF THE INVENTION**

In one aspect there is provided an electronic vapour provision system comprising:

a vaporiser for vaporising liquid for inhalation by a user of the electronic vapour provision system;
a power supply comprising a cell or battery for supplying power to the vaporiser
a container in which is contained a nicotine solution, wherein at least 60 wt% of the nicotine present in the solution is in protonated form, and wherein at least a portion of the container in contact with the nicotine solution is formed from polycarbonate or polypropylene.

In one aspect there is provided a cartomiser for an electronic vapour provision system, wherein the cartomiser comprises:

a container in which is contained a nicotine solution,
a wick material, and
a heating element for vaporising the nicotine;
wherein at least 60wt% of the nicotine present in the solution is in protonated form, and wherein at least a portion of the container in contact with the nicotine solution is formed from polycarbonate or polypropylene.

In one aspect there is provided a contained nicotine solution comprising

(i) a container; and
(ii) a nicotine solution contained within the container, wherein at least 5 wt% of the nicotine present in the solution is in protonated form,

wherein at least a portion of the container in contact with the nicotine solution is formed from polycarbonate or polypropylene.

In one aspect there is provided an electronic vapour provision system comprising:

a vaporiser for vaporising liquid for inhalation by a user of the electronic vapour provision system;
a power supply comprising a cell or battery for supplying power to the vaporiser
a container in which is contained a nicotine solution, wherein at least 5 wt% of the nicotine present in the solution is in protonated form, and wherein at least a portion of the container in contact with the nicotine solution is formed from polycarbonate or polypropylene.

In one aspect there is provided a process for stabilising a nicotine solution, the process comprising the steps of protonating the nicotine present in the solution such that at least 5 wt% of the nicotine present in the solution is in protonated form.

In one aspect there is provided use of protonation of nicotine for stabilising a nicotine solution.

**DETAILED DESCRIPTION**

As described above, the present disclosure relates to a container which may be used in an electronic vapour provision system, such as an e-cigarette. Throughout the following description the term "e-cigarette" is used; however, this term may be used interchangeably with electronic vapour provision system.

We have found that by protonating at least some of the nicotine present in a solution, the stability of the nicotine solution may be enhanced. We have found that solutions of nicotine when stored for significant periods suffer loss of nicotine content. By protonating at least a portion of the nicotine, and specifically at least 5 wt.% of the nicotine present, loss of the nicotine during storage is reduced. It has been found that loss of nicotine is particularly observed when nicotine solution is stored in contact with polycarbonate or polypropylene. These materials are desirable for use in -e-cigarettes due to their cost and their feel when held by a user. However, loss of nicotine may prohibit their use without the stabilisation of nicotine provided by the present invention.

As is understood by one skilled in the art, nicotine may exist in free base form, monoprotonated form or diprotonated form. The structures of each of these forms are given below.

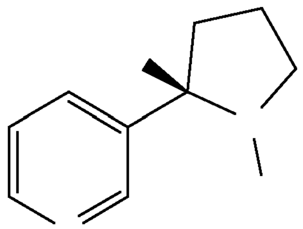
nicotine free base

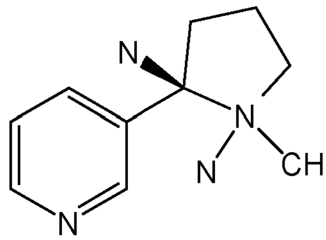
monoprotonated nicotine

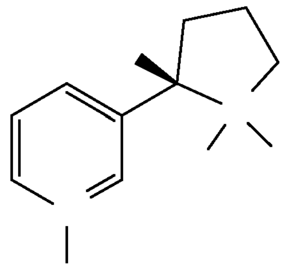
diprotonated nicotine

Reference in the specification to protonated form means both monoprotonated nicotine and diprotonated nicotine. Reference in the specification to amounts in the protonated form means the combined amount of monoprotonated nicotine and diprotonated nicotine.

For ease of reference, these and further aspects of the present invention are now discussed under appropriate section headings. However, the teachings under each section are not necessarily limited to each particular section.

The present invention provides a container in which is contained a nicotine solution, wherein at least 5 wt% of the nicotine present in the solution is in protonated form, wherein at least a portion of the container in contact with the nicotine solution is formed from polycarbonate or polypropylene.

The relevant amounts of nicotine which are present in the solution in protonated form are specified herein. These amounts may be readily calculated by one skilled in the art. Nicotine, 3-(1-methylpyrrolidin-2-yl) pyridine, is a diprotic base with pKa of 3.12 for the pyridine ring and 8.02 for the pyrrolidine ring. It can exist in pH-dependent protonated (mono- and di-) and non-protonated forms which have different bioavailability.

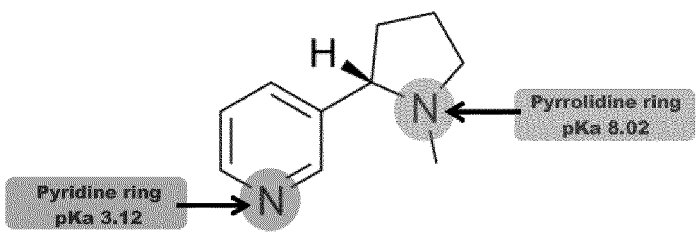


The distribution of protonated and non-protonated nicotine will vary at various pH increments.

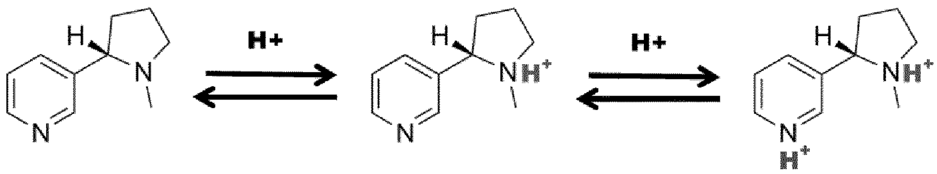


The fraction of non-protonated nicotine will be predominant at high pH levels whilst a decrease in the pH will see an increase of the fraction of protonated nicotine (mono- or di-depending on the pH). If the relative fraction of protonated nicotine and the total amount of nicotine in the sample are known, the absolute amount of protonated nicotine can be calculated.

The relative fraction of protonated nicotine in solution can be calculated by using the Henderson-Hasselbalch equation, which describes the pH as a derivation of the acid dissociation constant equation, and it is extensively employed in chemical and biological systems. Consider the following equilibrium:

$$B + H^{+} \rightleftarrows BH^{+}$$

The Henderson-Hasselbalch equation for this equilibrium is:

$$pH = pKa + \log \frac{[B]}{[BH+]}$$

Where [B] is the amount of non-protonated nicotine (i.e. free base), [BH+] the amount of protonated nicotine (i.e. conjugate acid) and pKa is the reference pKa value for the pyrrolidine ring nitrogen of nicotine (pKa=8.02). The relative fraction of protonated nicotine can be derived from the alpha value of the non-protonated nicotine calculated from the Henderson-Hasselbalch equation as:

$$\%\,protonated\ nicotine = 100 - \{\frac{\frac{[B]}{[BH+]}}{\left\{1 + \frac{[B]}{[BH+]}\right\}} * 100\}$$

Determination of pKa values of nicotine solutions may be was carried out using the basic approach described in "Spectroscopic investigations into the acid-base properties of nicotine at different temperatures", Peter M. Clayton, Carl A. Vas, Tam T. T. Bui, Alex F. Drake and Kevin McAdam, Anal. Methods, 2013,5, 81-88. This method is summarised below.

As will be understood by one skilled in the art, by polycarbonate it is meant a polymer containing the following repeating unit

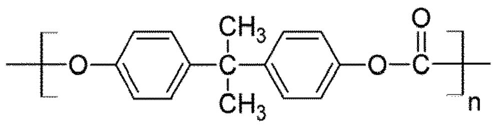

.

In one aspect at least a portion of the container in contact with the nicotine solution is formed from polycarbonate. In one aspect the majority of the container which is in contact with the nicotine solution is formed from polycarbonate. In one aspect all the container which is in contact with the nicotine solution is formed from polycarbonate. In one aspect the container is formed entirely from polycarbonate.

As will be understood by one skilled in the art, by polypropylene it is meant a polymer containing the following repeating unit

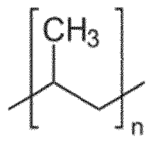.

In one aspect at least a portion of the container in contact with the nicotine solution is formed from polypropylene. In one aspect the majority of the container which is in contact with the nicotine solution is formed from polypropylene. In one aspect all the container which is in contact with the nicotine solution is formed from polypropylene. In one aspect the container is formed entirely from polypropylene.

In one aspect the majority of the container which is in contact with the nicotine solution is formed from polycarbonate, polypropylene or a combination thereof. In one aspect all the container which is in contact with the nicotine solution is formed from polycarbonate, polypropylene or a combination thereof. In one aspect the container is formed entirely from polycarbonate, polypropylene or a combination thereof.

As discussed herein, the container of the present invention is typically provided for the delivery of nicotine solution to or within an e-cigarette. The nicotine solution may be held within an e-cigarette or may be sold as a separate container for subsequent use with or in an e-cigarette. As understood by one skilled in the art, e-cigarettes typically contain a unit known as a cartomiser which comprises a reservoir of nicotine solution, a wick material and a heating element for vaporising the nicotine. In one aspect the container is a cartomiser or is part of a cartomiser. In one aspect the container is not a cartomiser or part of a cartomiser and is a container, such as a tank, bottle or the like, which may be used to deliver nicotine solution to or within an e-cigarette.

In one aspect the container is part of an e-cigarette. Therefore in a further aspect the present invention provides an electronic vapour provision system comprising: a vaporiser for vaporising liquid for inhalation by a user of the electronic vapour provision system; a power supply comprising a cell or battery for supplying power to the vaporiser; a container in which is contained a nicotine solution, wherein at least 5 wt% of the nicotine present in the solution is in protonated form, and wherein at least a portion of the container in contact with the nicotine solution is formed from polycarbonate or polypropylene.

As will be understood by one skilled in the art, contact may occur between the container and the nicotine solution by any means. Provided the body of the container is contacted with nicotine solution then the container and solution are in contact. It is envisaged that the nicotine solution could be 'free' in the sense that it is a liquid in direct contact with the walls of the container. In also envisaged, that the nicotine solution may be held within a matrix (such as a foam) and the foam is in contact with the body of the container.

As discussed herein, we have found that by protonating at least a portion of the nicotine, and specifically at least 5 wt.% of the nicotine present, loss of the nicotine during storage is reduced. In one aspect at least 10 wt% of the nicotine present in the solution is in protonated form. In one aspect at least 15 wt% of the nicotine present in the solution is in protonated form. In one aspect at least 20 wt% of the nicotine present in the solution is in protonated form. In one aspect at least 25 wt% of the nicotine present in the solution is in protonated form. In one aspect at least 30 wt% of the nicotine present in the solution is in protonated form. In one aspect at least 35 wt% of the nicotine present in the solution is in protonated form. In one aspect at least 40 wt% of the nicotine present in the solution is in protonated form. In one aspect at least 45 wt% of the nicotine present in the solution is in protonated form. In one aspect at least 50 wt% of the nicotine present in the solution is in protonated form. In one aspect at least 55 wt% of the nicotine present in the solution is in protonated form. In one aspect at least 60 wt% of the nicotine present in the solution is in protonated form. In one aspect at least 65 wt% of the nicotine present in the solution is in protonated form. In one aspect at least 70 wt% of the nicotine present in the solution is in protonated form. In one aspect at least 75 wt% of the nicotine present in the solution is in protonated form. In one aspect at least 80 wt% of the nicotine present in the solution is in protonated form. In one aspect at least 85 wt% of the nicotine present in the solution is in protonated form. In one aspect at least 90 wt% of the nicotine present in the solution is in protonated form. In one aspect at least 95 wt% of the nicotine present in the solution is in protonated form. In one aspect at least 99 wt% of the nicotine present in the solution is in protonated form.

The nicotine protonation may be provided in such a manner to achieve the desired degree of protonation of nicotine. In one aspect the nicotine is protonated by an organic acid. In one aspect the nicotine is protonated by a carboxylic acid. The carboxylic acid may be any suitable carboxylic acid. In one aspect the nicotine is protonated by a mono-carboxylic acid.

In one aspect the nicotine is protonated by an acid selected from the group consisting of acetic acid, lactic acid, formic acid, citric acid, benzoic acid, pyruvic acid, levulinic acid, succinic acid, tartaric acid, oleic acid, sorbic acid, propionic acid, phenylacetic acid, and mixtures thereof.

In one aspect the nicotine is protonated by an acid selected from the group consisting of benzoic acid, levulinic acid, and mixtures thereof. In one aspect the nicotine is protonated by levulinic acid. In one aspect the nicotine is protonated by benzoic acid. In one aspect the nicotine is protonated by a mixture of levulinic acid and benzoic acid.

Nicotine may exist in free base form, monoprotonated form or diprotonated form.

As discussed herein we have found that by protonating at least some of the nicotine present in a solution, the stability of the nicotine solution may be enhanced. We have found that solutions of nicotine when stored for significant periods suffer loss of nicotine content. Although problems of stability are particularly observed when nicotine solution is stored in contact with polycarbonate or polypropylene, the problems are not exclusive to those materials. The present invention therefore provides a novel process for stabilising a nicotine solution. The present invention provides a process for stabilising a nicotine solution, the process comprising the steps of protonating the nicotine present in the solution such that at least 5 wt% of the nicotine present in the solution is in protonated form.

In the process of the present invention the nicotine solution may be in contact with polycarbonate or polypropylene.

We have found that extended periods of storage are possible with the practice of the present invention. In one aspect the process provides for storage of the nicotine solution in contact with polycarbonate or polypropylene for a period of at least 7 days. In one aspect the process provides for storage of the nicotine solution in contact with polycarbonate or polypropylene for a period of at least 14 days. In one aspect the process provides for storage of the nicotine solution in contact with polycarbonate or polypropylene for a period of at least 21 days. In one aspect the process provides for storage of the nicotine solution in contact with polycarbonate or polypropylene for a period of at least 28 days. In one aspect the process provides for storage of the nicotine solution in contact with polycarbonate or polypropylene for a period of at least 2 months. In one aspect the process provides for storage of the nicotine solution in contact with polycarbonate or polypropylene for a period of at least 3 months. In one aspect the process provides for storage of the nicotine solution in contact with polycarbonate or polypropylene for a period of at least 4 months. In one aspect the process provides for storage of the nicotine solution in contact with polycarbonate or polypropylene for a period of at least 5 months. In one aspect the process provides for storage of the nicotine solution in contact with polycarbonate or polypropylene for a period of at least 6 months.

The present invention further provides a novel use for stabilising a nicotine solution. In one aspect the present invention provides use of protonation of nicotine for stabilising a nicotine solution. In one aspect the present invention provides use of protonation of nicotine for improving storage stability of a nicotine solution. In one aspect the present invention provides use of protonation of nicotine for reducing evaporative loss of nicotine from a nicotine solution.

In one aspect the present invention provides use of protonated nicotine for stabilising a solution containing nicotine free base. In one aspect the present invention provides use of protonated nicotine for improving storage stability of a solution containing nicotine free base. In one aspect the present invention provides use of protonated nicotine for reducing evaporative loss of nicotine from a nicotine free base solution. It will be understood that by 'nicotine free base solution' it may be meant a solution containing nicotine free base and protonated nicotine in an amount as described herein.

In one aspect the present invention provides use of an acid for stabilising a nicotine solution. In one aspect the present invention provides use of an acid for improving storage stability of a nicotine solution. In one aspect of use in accordance with the present invention the acid is an organic acid. In one aspect of use in accordance with the present invention the acid is a carboxylic acid. In one aspect of use in accordance with the present invention the acid is a monocarboxylic acid. In one aspect of use in accordance with the present invention the acid is selected from the group consisting of acetic acid, lactic acid, formic acid, citric acid, benzoic acid, pyruvic acid, levulinic acid, succinic acid, tartaric acid, oleic acid, sorbic acid, propionic acid, phenylacetic acid, and mixtures thereof. In one aspect of use in accordance with the present invention the acid is selected from the group consisting of benzoic acid, levulinic acid, and mixtures thereof. In one aspect of use in accordance with the present invention the acid is levulinic acid. In one aspect of use in accordance with the present invention the acid is benzoic acid. In one aspect of use in accordance with the present invention the acid is a mixture of levulinic acid and benzoic acid.

In one aspect the present invention provides use of an acid for stabilising a nicotine solution, wherein the acid is selected from the group consisting of benzoic acid, levulinic acid, and mixtures thereof. In one aspect the present invention provides use of an acid for improving storage stability of a nicotine solution, wherein the acid is selected from the group consisting of benzoic acid, levulinic acid, and mixtures thereof. In one aspect the present invention provides use of an acid for reducing evaporative loss of nicotine from a nicotine solution, wherein the acid is selected from the group consisting of benzoic acid, levulinic acid, and mixtures thereof.

In the use aspects of the present invention at least 5 wt% of the nicotine present in the nicotine solution may be in protonated form.

In one aspect the present invention provides use of protonation of nicotine for stabilising a nicotine solution with respect to polycarbonate or polypropylene. In one aspect the present invention provides use of protonation of nicotine for improving storage stability of a nicotine solution with respect to polycarbonate or polypropylene.

The invention will now be described with reference to the following non-limiting examples and with reference to the accompanying figure in which:

Figure 1 shows a graph.

## Examples

### Example 1 - Determination of pKa Values

The determination of pKa values of nicotine in glycerol/water systems was carried out using the basic approach described in "Spectroscopic investigations into the acid-base properties of nicotine at different temperatures", Peter M. Clayton, Carl A. Vas, Tam T. T. Bui, Alex F. Drake and Kevin McAdam, ·Anal. Methods, 2013,5, 81-88, and summarised below. Because the system is predominately non-aqueous the parameter $p_sK_{a2}$ was measured, where subscript s refers to the solvent composition in this largely non-aqueous system, and subscript 2 refers to the $pK_a$ value of the pyrrolidyl nitrogen.

Further information on the determination of pKa values of nicotine in e-cigarette solutions is provided in "Use of chiroptical spectroscopy to determine the ionisation status of (S)-nicotine in e-cigarette formulations and snus", Clayton et al, ST 49, CORESTA Congress, Québec City, Canada, 12-16 October 2014 (available at http://www.bat-science.com/groupms/sites/BAT_9GVJXS.nsf/vwPagesWebLive/DO9PVC3G/$FILE/CORES TA_PC_2014.pdf)

A range of glycerol/water/nicotine solutions were prepared, with the water concentration fixed at 9%, the nicotine concentration varying from 30μg/ml to 3mg/ml; and the glycerol content comprising the remainder of the solutions.

Simultaneous UV & CD spectra of glycerol/s-nicotine/water solutions were measured on the Applied Photophysics Ltd (Leatherhead, UK) Chiracsan Plus spectrometer. The UV absorbance & CD spectra were measured between 300-200 nm region, with various pathlengths depending upon the nicotine concentration of the solution - 10mm, 5mm, 2mm, 1mm, 0.5mm, 0.1mm and 0.01mm pathlengths. The instrument was flushed continuously with pure evaporated nitrogen throughout the measurements. Throughout measurements spectra were recorded with a 0.5 nm step size, a 1s measurement time-per-point and a spectral bandwidth of 2 nm. Where possible, all CD spectra were smoothed with a window factor of 4 using the Savitzky-Golay method for better presentation.

Solutions of S-Nicotine in glycerol/water were pH titrated at 23°C. The pH of these solutions was raised towards alkaline by adding small aliquots of NaOH (~pH10) and then lowered to pH2 by adding small aliquots of HCl. A series of 0.1M, 0.5M, 1M, 5M and 10M of HCl and NaOH solutions were used during the pH titration. pHs were measured at 23°C using a Corning pH105 pH meter with a RMS pH electrode. The $p_sK_{a2}$ values changed systematically with nicotine concentration (Figure 1) and therefore values for $p_sK_{a2}$ were calculated at each nicotine concentration level (Table 1). Due to the viscosity of the solutions, and the optical density in the CD spectra of the high nicotine concentration solutions, very small path-length cells were required for nicotine concentrations above 3mg/ml. Satisfactory sample preparation and spectroscopy could not be achieved with the necessary small cells at these concentrations, and therefore the $p_sK_{a2}$ at higher concentrations were calculated from a regression fit to Figure 1.

*Table 1:* $p_sK_{a2}$ values measured at various nicotine concentrations in a 9% water, nicotine/glycerol system.

| $p_sK_{a2}$ | **conc** (g/L) | **conc** (mM) | $\log_{10}$ **[conc]** |
|---|---|---|---|
| 7.49 | 0.03 | 0.185 | -0.732 |
| 7.34 | 0.06 | 0.370 | -0.431 |
| 7.30 | 0.3 | 1.85 | 0.268 |
| 7.27 | 0.6 | 3.70 | 0.569 |
| 7.25 | 3 | 18.53 | 1.268 |

Curve fitting, using the equation *$y= 0.0233e^{(-(log10[nicotine])/0.325)} + 7.26$ provided a $p_sK_{a2}$ value of 7.26 at 30 mg/ml nicotine concentration. Use of this $p_sK_{a2}$ value with the* Henderson-Hasselbalch equation allows calculation of the degree of nicotine protonationm at any pH value.

### Example 2 - Determination of pH and % Protonation

The materials were formulated as described and the pH determined as described in Example 1. Based on the pKa of 7.26 determined in Example 1, the percentage of nicotine that was protonated was calculated using the Henderson-Hasselbalch equation. The results obtained are tabulated below.

| | Formation composition (% w/w) | | | | | | Flavours | Average pH | Average Temp ($^0C$) | % protonated |
|---|---|---|---|---|---|---|---|---|---|---|
| Formulation | Nicotine | Glycerol | Water | PG + flavour level | Benzoic acid level | Levulinic acid level | | | | |
| 4% w/w Nicotine in glycerol/water | 4 | 87 | 9 | 0 | 0.00 | 0.00 | not present | 9.21 | 22.1 | 1.1 |
| 4% w/w Nicotine in glycerol/water/PG | 4 | 62.00 | 9 | 25 | 0.00 | 0.00 | not present | 9.18 | 21.8 | 1.2 |
| 4% w/w Nicotine + Cherry flavour | 4 | 62.00 | 9 | 25 | 0.00 | 0.00 | present | 8.23 | 22.7 | 9.7 |
| 4% w/w Nicotine + Cherry flavour + 0.4M Benzoic Acid | 4 | 60.80 | 9 | 25 | 1.20 | 0.00 | present | 7.16 | 21.0 | 55.7 |
| 4% w/w Nicotine + Cherry flavour + 0.4M Levulinic Acid | 4 | 60.85 | 9 | 25 | 0.00 | 1.15 | present | 6.99 | 21.0 | 65.1 |
| | | | | | | | | | | |
| 1.8% w/w Nicotine + glycerol/water | 1.86 | 89.2 | 9 | 0 | 0.00 | 0.00 | not present | 9.32 | 22.2 | 0.9 |
| 1.8% w/w Nicotine in glycerol/water/PG | 1.86 | 42.2 | 25 | 25 | 0.00 | 0.00 | not present | 9.21 | 22.2 | 1.1 |
| 1.8% w/w Nicotine + Cherry flavour | 1.86 | 42.2 | 25 | 25 | 0.00 | 0.00 | present | 8.14 | 21.2 | 11.6 |
| 1.8% w/w Nicotine + Cherry flavour + 0.4M Benzoic Acid | 1.86 | 47.59 | 25 | 25 | 0.55 | 0.00 | present | 7.34 | 21.3 | 45.4 |
| 1.8% w/w Nicotine + Cherry + 0.4M Levulinic Acid | 1.86 | 47.62 | 25 | 25 | 0.00 | 0.52 | present | 7.08 | 21.2 | 60.2 |
| | | | | | | | | | | |
| 4% w/w Nicotine in glycerol/water | 4 | 87 | 9 | 0 | 0.00 | 0.00 | not present | 9.21 | 22.1 | 1.1 |
| 4% w/w Nicotine in glycerol/water/PG | 4 | 52 | 9 | 35 | 0.00 | 0.00 | not present | 9.11 | 22.4 | 1.4 |
| 4% w/w Nicotine + Menthol | 4 | 50.5 | 9 | 36.5 | 0.00 | 0.00 | present | 9.36 | 22.3 | 0.8 |

(continued)

| | Formation composition (% w/w) | | | | | | Flavours | Average pH | Average Temp ($^0C$) | % protonated |
|---|---|---|---|---|---|---|---|---|---|---|
| Formulation | Nicotine | Glycerol | Water | PG + flavour level | Benzoic acid level | Levulinic acid level | | | | |
| 4% w/w Nicotine + Menthol + 0.4M Benzoic Acid | 4 | 49.3 | 9 | 36.5 | 1.20 | 0.00 | present | 6.95 | 21.1 | 67.1 |
| 4% w/w Nicotine + Menthol + 0.4M Levulinic Acid | 4 | 49.35 | 9 | 36.5 | 0.00 | 1.15 | present | 6.81 | 21.1 | 73.8 |
| | | | | | | | | | | |
| 1.8% w/w Nicotine in glycerol/water | 1.86 | 89.2 | 9 | 0 | 0.00 | 0.00 | not present | 9.32 | 22.2 | 0.9 |
| 1.8% w/w Nicotine in glycerol/water/PG | 1.86 | 38.14 | 25 | 35 | 0.00 | 0.00 | not present | 9.08 | 22.7 | 1.5 |
| 1.8% w/w Nicotine + Menthol | 1.86 | 36.64 | 25 | 36.5 | 0.00 | 0.00 | present | 9.07 | 21.2 | 1.5 |
| 1.8% w/w Nicotine + Menthol + 0.4M Benzoic Acid | 1.86 | 36.09 | 25 | 36.5 | 0.55 | 0.00 | present | 7.13 | 21.1 | 57.4 |
| 1.8% w/w Nicotine + Menthol + 0.4M Levulinic Acid | 1.86 | 36.145 | 25 | 36.5 | 0.00 | 0.50 | present | 6.95 | 21.4 | 67.1 |
| | | | | | | | | | | |
| 1.8% Nicotine, 25% Water, 25% PG with Tobacco flavour "A" | 1.8 | 48.2 | 25 | 25 | 0.00 | 0.00 | present | 8.5 | | 5.4 |
| 1.8% Nicotine, 25% Water, 25% PG with Tobacco flavour "A", 0.3 Molar equivalent Benzoic | 1.8 | 47.79 | 25 | 25 | 0.41 | 0.00 | present | 7.4 | | 42.0 |
| 1.8% Nicotine, 25% Water, 25% PG with Tobacco flavour "A" 0.75 Molar equivalent Benzoic | 1.8 | 47.17 | 25 | 25 | 1.03 | 0.00 | present | 6.8 | | 74.3 |

N/N-H: Nicotine/Protonated nicotine
PG: propylene glycol

**Example 3 - Storage stability of Nicotine in solutions exposed to PP and PC**

The absorption of nicotine by a number of materials suitable for use in e-cigarettes was studied. The objective of this study was to determine whether there was any absorption of nicotine from a liquid formulation into a variety of materials occurring over time. 5g of a nicotine solution comprising 3.7 wt% nicotine, 9% water and 87.3% glycerol was loaded into 40ml amber glass vials. Pieces of polypropylene and polycarbonate were added to solutions (except for control samples), the vials were sealed with screw caps and stored in an at ambient temperatures or at 40°C in an oven for a period of eight weeks. Sampling was performed on day 1, then at 1 week, 2 weeks, 4 weeks and 8 weeks.

An accurately weighed aliquot of solution was removed from the vials using a Pasteur pipette and diluted with water (~40mg sample in 1 ml). The final solution weights were also recorded. Analysis for nicotine was performed by LC-UV. using a Waters Acquity LC system incorporating a Diode Array detector.

All analyses were performed using a 1000ppm external nicotine standard prepared in water. Linearity of analysis of nicotine was checked at each time-point with 500ppm, 1000ppm and 2000ppm standards.

Time-Point Time in Storage Analysis Date

| | |
|---|---|
| T = 0 | 1 day |
| T = 1 | 1 week |
| T = 2 | 2 weeks |
| T = 4 | 4 weeks |
| T = 8 | 8 weeks |

The findings are reported below in tabulated format.

| Container | Temp | | T = 0 | T = 1 | T = 2 | T = 3 | T = 4 |
|---|---|---|---|---|---|---|---|
| Control | Ambient | wt% Nicotine | 4.16 | 4.17 | 4.10 | 4.06 | 3.84 |
| | | % loss | | 0.25 | -1.51 | -2.39 | -7.71 |
| Control | 40°C | wt% Nicotine | 4.16 | 4.18 | 4.05 | 4.10 | 3.97 |
| | | % loss | | 0.41 | -2.62 | -1.38 | -4.68 |
| PP | Ambient | wt% Nicotine | 4.20 | 4.16 | 4.03 | 4.07 | 3.61 |
| | | % loss | | -0.92 | -3.98 | -3.18 | -14.05 |
| PP | 40°C | wt% Nicotine | 4.27 | 4.09 | 3.96 | 3.94 | 3.70 |
| | | % loss | | -4.24 | -7.35 | -7.80 | -13.37 |
| PC | Ambient | wt% Nicotine | 4.10 | 4.10 | 4.12 | 4.01 | 3.58 |
| | | % loss | | 0.03 | 0.60 | -2.15 | -12.57 |
| PC | 40°C | wt% Nicotine | 4.20 | 4.09 | 4.08 | 4.07 | 3.61 |
| | | % loss | | -2.66 | -2.84 | -3.16 | -14.10 |

As can be seen from the above Table, polypropylene (PP) and polycarbonate (PC) both of which are desirable materials for use in e-cigarettes were found to result in significant loss of nicotine when stored in contact with a nicotine solution containing nicotine only in free base form.

**Example 4 - Storage Stability of Protonated Nicotine in e-cigarettes**

The effect on storage stability of protonating nicotine was studied by examining 3 nicotine solutions loaded into

cartomizer e-cigarettes ("Device") containing PP and PC. The three nicotine solutions were an acid free nicotine solution and two protonated solutions, one protonated with 1.0 Molar equivalent levulinic acid and one protonated with 1.0 Molar equivalent benzoic acid. For each of the formulations manufactured a 2.5% w/w nicotine amount was used, together with 9% water and sufficient glycerol to make the solution to 100%. The stability protocol incorporated filling a series of e-cigarettes for each formulation, as well as loading a number of sealed glass vials (used as control samples) to understand the source of any observed nicotine losses.

Over the duration of the study samples were stored at 25deg Celsius/ 60% relative humidity and 40deg Celsius/ 75% relative humidity for a total of 9 weeks, data was collected at time points 1, 5 and 9 weeks. During the study time the e-cigarette solution was in contact with the internal materials of the cartomizer (including PP and PC) in a manner reflecting real-world use. In the table below, T0 = 1 week, T4 = 5 Weeks and T8 = 9 Weeks.

At each of the above time points the amount of nicotine present within formulation was determined as follows.

For analysis of e-liquids: approximately 100 μl liquid was extracted into 20 ml extraction solvent and analysed as described for aerosol determination.

The e-cigarettes were puffed on a 20-channel linear smoking machine (SM450) compliant with ISO 3308, but using the following puffing parameters: 80ml puff volume, 3 second puff duration and 30 second interpuff interval. Each port of the smoking engine was fitted with a holder containing a Cambridge filter (CF) pad to trap particulate matter. After puffing TPM was determined as the weight difference of the CF before and after puffing, in accordance with ISO 4387. The CF pad containing trapped aerosol was extracted into 20 ml high purity propan-2-ol containing appropriate internal standards. Nicotine and water were determined by GC analysis containing combined FID/TCD detectors.

The data below reports the nicotine levels measured.

| | T0 (25°C/60%RH) | |
|---|---|---|
| | Nicotine (%w/w) | |
| Formulation | Vial | Device |
| Standard | 2.36 | 2.11 |
| + Levulinic Acid | 2.35 | 2.29 |
| + Benzoic Acid | 2.37 | 2.31 |

| | T4 (25°C/60%RH) | |
|---|---|---|
| | Nicotine (%w/w) | |
| Formulation | Vial | Device |
| Standard | 2.49 | 1.48 |
| + Levulinic Acid | 2.51 | 2.03 |
| + Benzoic Acid | 2.53 | 2.12 |

| | T4 (40°C/75%RH) | |
|---|---|---|
| | Nicotine (%w/w) | |
| Formulation | Vial | Device |
| Standard | 2.49 | 0.94 |
| + Levulinic Acid | 2.5 | 1.68 |
| + Benzoic Acid | 2.52 | 1.8 |

| | T8 (25°C/60%RH) | |
|---|---|---|
| | Nicotine (%w/w) | |
| Formulation | Vial | Device |
| Standard | 2.42 | 0.94 |
| + Levulinic Acid | 2.4 | 1.55 |
| + Benzoic Acid | 2.43 | 1.7 |

| | T8 (40°C/75%RH) | |
|---|---|---|
| | Nicotine (%w/w) | |
| Formulation | Vial | Device |
| Standard | 2.41 | 0.18 |
| + Levulinic Acid | 2.44 | 0.96 |
| + Benzoic Acid | 2.44 | 1.2 |

In further aspects the present invention provides the following:

1. A contained nicotine solution comprising

(i) a container; and
(ii) a nicotine solution contained within the container, wherein at least 5 wt% of the nicotine present in the solution is in protonated form,

wherein at least a portion of the container in contact with the nicotine solution is formed from polycarbonate or polypropylene.

2. A contained nicotine solution according to paragraph 1 wherein the majority of the container in contact with the nicotine solution is formed from polycarbonate or polypropylene.

3. A contained nicotine solution according to paragraph 1 or 2 wherein at least a portion of the container in contact with the nicotine solution is formed from polycarbonate.

4. A contained nicotine solution according to paragraph 1, 2 or 3 wherein at least a portion of the container in contact with the nicotine solution is formed from polypropylene.

5. A contained nicotine solution according to any one of paragraphs 1 to 4 wherein the container is a cartomiser or is part of a cartomiser.

6. A contained nicotine solution according to any one of paragraphs 1 to 5 wherein at least 20 wt% of the nicotine present in the solution is in protonated form.

7. A contained nicotine solution according to any one of paragraphs 1 to 6 wherein at least 40 wt% of the nicotine present in the solution is in protonated form.

8. A contained nicotine solution according to any one of paragraphs 1 to 7 wherein the nicotine is protonated by an acid selected from the group consisting of acetic acid, lactic acid, formic acid, citric acid, benzoic acid, pyruvic acid, levulinic acid, succinic acid, tartaric acid, oleic acid, sorbic acid, propionic acid, phenylacetic acid, and mixtures thereof.

9. A contained nicotine solution according to any one of paragraphs 1 to 8 wherein the nicotine is protonated by levulinic acid.

10. A contained nicotine solution according to any one of paragraphs 1 to 9 wherein the nicotine is protonated by benzoic acid.

11. An electronic vapour provision system comprising:

a vaporiser for vaporising liquid for inhalation by a user of the electronic vapour provision system;
a power supply comprising a cell or battery for supplying power to the vaporiser
a container in which is contained a nicotine solution, wherein at least 5 wt% of the nicotine present in the solution is in protonated form, and wherein at least a portion of the container in contact with the nicotine solution is formed from polycarbonate or polypropylene.

12. An electronic vapour provision system according to paragraph 11 wherein the container or nicotine solution is as defined in any one of paragraphs 2 to 10.

13. A process for stabilising a nicotine solution, the process comprising the steps of protonating the nicotine present in the solution such that at least 5 wt% of the nicotine present in the solution is in protonated form.

14. A process according to paragraph 13 wherein the nicotine solution is contacted with polycarbonate or polypropylene.

15. A process according to paragraph 13 or 14 wherein the nicotine solution is stored in contact with polycarbonate or polypropylene for a period of at least 7 days.

16. Use of protonation of nicotine for stabilising a nicotine solution.

17. Use according to paragraph 16 for improving storage stability of the nicotine solution.

18. Use according to paragraph 16 or 17 wherein at least 5 wt% of the nicotine present in the nicotine solution is in protonated form.

19. Use according to any one of paragraphs 16 to 18 wherein the nicotine solution is stabilised with respect to polycarbonate or polypropylene.

20. Use according to any one of paragraphs 16 to 19 wherein evaporative loss is reduced.

21. A process or use according to any one of paragraphs 13 to 20 wherein the nicotine is protonated by an acid selected from the group consisting of acetic acid, lactic acid, formic acid, citric acid, benzoic acid, pyruvic acid, levulinic acid, succinic acid, tartaric acid, oleic acid, sorbic acid, propionic acid, phenylacetic acid, and mixtures thereof.

22. A process or use according to any one of paragraphs 13 to 21 wherein the nicotine is protonated by levulinic acid.

23. A process or use according to any one of paragraphs 13 to 22 wherein the nicotine is protonated by benzoic acid.

Various modifications and variations of the present invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in chemistry or related fields are intended to be within the scope of the following claims.

## Claims

1. A process for stabilising a nicotine solution, the process comprising the steps of protonating the nicotine present in the solution such that at least 5 wt% of the nicotine present in the solution is in protonated form.

2. A process according to claim 1 wherein the nicotine solution is stabilised with respect to polycarbonate or polypropylene.

3. A process according to claim 2 wherein the nicotine solution is in a container wherein at least a portion of the container in contact with the nicotine solution is formed from polycarbonate or polypropylene.

4. Use of protonation of nicotine for reducing evaporative loss of nicotine from a nicotine solution.

5. Use according to claim 4 wherein at least 5 wt% of the nicotine present in the nicotine solution is in protonated form.

6. Use according to claim 4 or 5 wherein the nicotine solution is in a container wherein at least a portion of the container in contact with the nicotine solution is formed from polycarbonate or polypropylene.

7. Use of protonation of nicotine for reducing evaporative loss of nicotine in an electronic vapour provision system comprising:

   a vaporiser for vaporising liquid for inhalation by a user of the electronic vapour provision system;
   a power supply comprising a cell or battery for supplying power to the vaporiser
   a container in which is contained the nicotine solution, wherein at least 5 wt% of the nicotine present in the solution is in protonated form, and wherein at least a portion of the container in contact with the nicotine solution is formed from polycarbonate or polypropylene.

8. A process or use according to claim 3, 6 or 7 wherein the majority of the container in contact with the nicotine solution is formed from polycarbonate.

9. A process or use according to claim 3, 6 or 7 wherein the majority of the container in contact with the nicotine solution is formed from polypropylene.

10. A process or use according to any one of claims 1 to 9 wherein at least 20 wt% of the nicotine present in the solution is in protonated form, such as at least 40 wt% of the nicotine present in the solution is in protonated form, such as at least 70 wt% of the nicotine present in the solution is in protonated form, such as at least 80 wt% of the nicotine present in the solution is in protonated form.

11. A process or use according to any one of claims 1 to 10 wherein the nicotine is protonated by an acid selected from the group consisting of acetic acid, lactic acid, formic acid, citric acid, benzoic acid, pyruvic acid, levulinic acid, succinic acid, tartaric acid, oleic acid, sorbic acid, propionic acid, phenylacetic acid, and mixtures thereof.

12. A process or use according to any one of claims 1 to 11 wherein the nicotine is protonated by levulinic acid.

13. A process or use according to any one of claims 1 to 11 wherein the nicotine is protonated by benzoic acid.

14. A process or use according to any one of claims 1 to 11 wherein the nicotine is protonated by lactic acid.

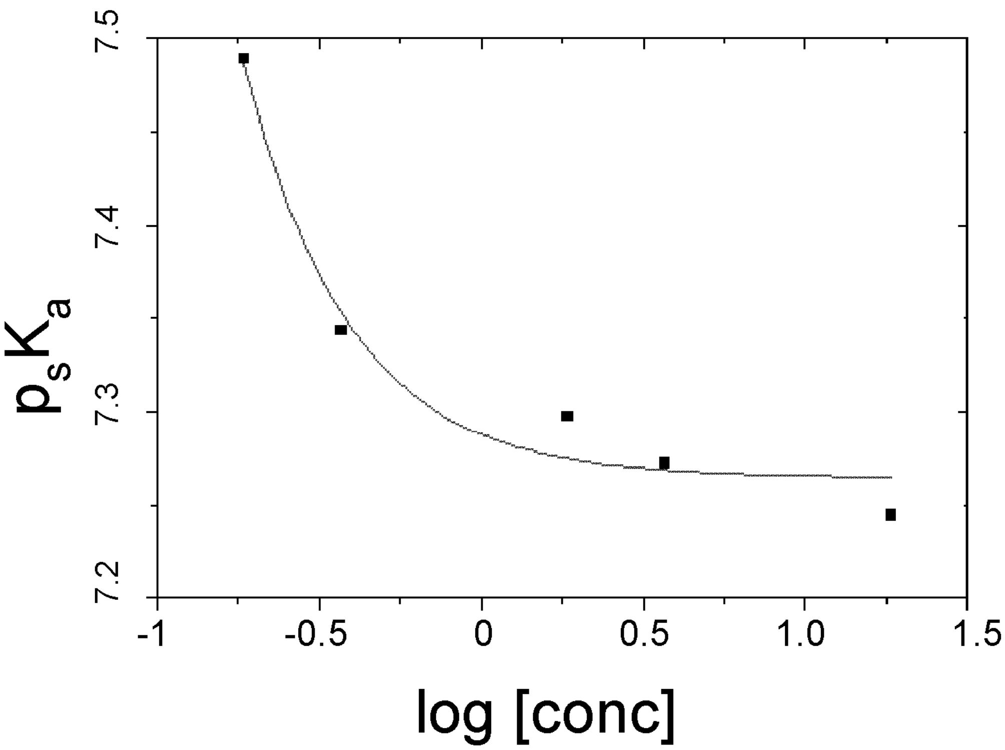


*Figure 1: Variation of* $p_sK_{a2}$ with nicotine concentration

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **PETER M. CLAYTON ; CARL A. VAS ; TAM T. T. BUI ; ALEX F. DRAKE ; KEVIN MCADAM.** pectroscopic investigations into the acid-base properties of nicotine at different temperatures. *Anal. Methods,* 2013, vol. 5, 81-88
- **PETER M. CLAYTON ; CARL A. VAS ; TAM T. T. BUI ; ALEX F. DRAKE ; KEVIN MCADAM.** Spectroscopic investigations into the acid-base properties of nicotine at different temperatures. ·*Anal. Methods,* 2013, vol. 5, 81-88
- **CLAYTON et al.** Use of chiroptical spectroscopy to determine the ionisation status of (S)-nicotine in e-cigarette formulations and snus. *ST 49, CORESTA Congress, Québec City, Canada,* 12 October 2014, http://www.bat-science.com/groupms/sites/BAT_9GVJXS.nsf/vwPagesWebLive/DO9PVC3G/$FILE/CORESTA_PC_2014.pdf